# FASCICULE DE BREVET EUROPEEN

(11) **EP 2 072 035 B1**
(45) Date de publication et mention de la délivrance du brevet: **17.09.2014**
(21) Numéro de dépôt: 08172449.4
(22) Date de dépôt: 19.12.2008
(51) Int. Cl.: A61K 8/41, A61Q 5/08

(54) **Procédé d'éclaircissement de fibres kératiniques humaines mettant en oeuvre une composition anhydre et une amine organique particuliere et dispositif appropré**
Verfahren zum Aufhellen menschlicher Keratinfasern mit einer wasserfreien Zusammensetzung und einem speziellen organischen Amin sowie eine Vorrichtung hierfür
Process for lightening human keratin fibres using an anhydrous composition and a particular organic amine, and device suitable therefore

(30) Priorité: 21.12.2007 FR 0760274
(43) Date de publication de la demande: 24.06.2009
(73) Titulaire: L'Oréal, 75008 Paris (FR)
(72) Inventeur: Hercouet, Leïla, 93360 Neuilly Plaisance (FR); Lagrange, Alain, 77700 Coupvray (FR)
(74) Mandataire: Wattremez, Catherine

(56) Documents cités:
- EP-A- 1 291 006
- EP-A- 1 438 951
- EP-A- 1 598 052

## Description

La présente invention a pour objet un procédé d'éclaircissement de fibres kératiniques humaines mettant en jeu une composition anhydre (A) comprenant un ou plusieurs corps gras et un ou plusieurs tensioactifs, une composition (B) comprenant une ou plusieurs amines organiques dont le pKb est inférieur à 12 à 25°C et une composition (C) comprenant un ou plusieurs agents oxydants.

Elle concerne enfin un dispositif à plusieurs compartiments dont l'un au moins comprend la composition cosmétique anhydre (A) précitée, un autre la composition (B) et un dernier la composition oxydante (C).

Les procédés d'éclaircissement des fibres kératiniques humaines consistent à employer une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que le procédé d'éclaircissement est mis en oeuvre dans des conditions alcalines et que l'agent alcalin le plus couramment utilisé est l'ammoniaque. L'ammoniaque est particulièrement avantageux dans ce type de procédé. En effet, il permet d'ajuster le pH de la composition à un pH alcalin pour permettre la dégradation de l'agent oxydant. Mais cet agent provoque également un gonflement de la fibre kératinique, avec une ouverture des écailles, ce qui favorise la pénétration de l'oxydant à l'intérieur de la fibre et donc augmente l'efficacité de la réaction.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

EP-A-1 438 951 décrit des compositions cosmétiques anhydres contenant un corps gras (myristate d'isopropyle) et un tensioactif. Une composition oxydante est mélangée avec la composition anhydre pour former la composition de décoloration.

EP-A-1 598 052 divulgue des compositions d'éclaircissement et de coloration de fibres kératiniques contenant une composition anhydre qui contient un corps gras, une composition contenant des corps gras, des tensioactifs et des colorants et une composition oxydante.

EP-A-1 291 006 décrit des compositions cosmétiques aqueuses pour l'éclaircissement et la coloration de fibres kératiniques contenant des corps gras, des tensioactifs et des amines organiques.

L'un des objectifs de la présente invention est de proposer des procédés d'éclaircissement des fibres kératiniques humaines qui ne présentent pas les inconvénients de ceux mis en oeuvre avec les compositions existantes, inconvénients causés par la présence de teneurs importantes en ammoniaque, mais qui restent au moins aussi efficaces sur le plan de l'éclaircissement et de l'homogénéité de ce dernier.

Ces buts et d'autres sont atteints par la présente invention qui a donc pour objet un procédé d'éclaircissement de fibres kératiniques humaines dans lequel on met en oeuvre :
a) une composition anhydre (A) comprenant un ou plusieurs corps gras et un ou plusieurs tensioactifs ;
b) une composition (B) comprenant une ou plusieurs amines organiques dont le pKb est inférieur à 12 à 25°C ;
c) une composition (C) comprenant un ou plusieurs agents oxydants.

Elle concerne également un dispositif à plusieurs compartiments comprenant dans l'un d'eux une composition anhydre (A), dans un autre une composition (B) et dans un autre, une composition (C) comprenant un ou plusieurs agents oxydants.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

La composition cosmétique anhydre (A) présente plus particulièrement une teneur en eau inférieure à 5% en poids, de préférence inférieure à 2% en poids, et de manière encore plus particulière inférieure à 1% en poids, par rapport au poids de ladite composition. II est à noter que l'eau peut aussi se trouver sous forme d'eau liée, comme l'eau de cristallisation des sels ou des traces d'eau absorbée par les matières premières utilisées dans la réalisation des compositions selon l'invention.

En outre, cette composition ne comprend pas de colorant direct ou de précurseur de colorant d'oxydation (bases et coupleurs) utilisés habituellement pour la coloration des fibres kératiniques humaines ou bien, si elle en comprend, leur teneur totale ne dépasse pas 0,005 % en poids par rapport au poids de la composition anhydre et de la composition aqueuse comprenant l'agent oxydant. En effet, à une telle teneur, seule la composition serait teintée, c'est-à-dire qu'on n'observerait pas d'effet de coloration des fibres kératiniques.

De préférence, la composition anhydre cosmétique ne comprend pas de bases d'oxydation telle que les paraphénylènediamines, les bis-phénylalkylènediamines, les para-aminophénols, les ortho-aminophénols, les bases hétérocycliques et leurs sels d'addition avec un acide. Elle ne comprend également de préférence, pas de coupleurs comme par exemple les méta-phénylènediamines, les méta-aminophénols, les méta-diphénols, les coupleurs naphtaléniques, les coupleurs hétérocycliques ainsi que leurs sels d'addition. Enfin, la composition anhydre cosmétique ne comprend de préférence pas de colorants directs non ioniques ou ioniques (cationiques ou anioniques) comme par exemple les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Ainsi que cela a été mentionné, la composition cosmétique anhydre selon l'invention comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0.1%). En outre, les corps gras sont solubles dans les solvants organiques dans les mêmes conditions de température et de pression, comme par exemple le chloroforme, l'éthanol ou le benzène.

Selon l'invention, les corps gras sont choisis parmi les composés liquides ou pâteux à température ambiante et à pression atmosphérique.

Plus particulièrement, les corps gras sont choisis parmi les alcanes, les alcools gras, les acides gras, les esters d'acide gras, les esters d'alcool gras, les huiles minérales, végétales, animales ou synthétiques, les silicones, les cires.

II est rappelé qu'au sens de l'invention, les alcools, esters et acides gras présentent plus particulièrement au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes, ces derniers comprennent de 6 à 30 atomes de carbone, sont linéaires. A titre d'exemple, on peut citer l'hexane, le dodécane.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam^{®} ; les isoparaffines comme l'isohexadécane et l'isodécane.
- les alcools gras sont saturés ou insaturés, linéaires ou ramifiés, et comportent de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

La cire ou les cires sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras peuvent être saturés ou insaturés et comportent de 6 à 30 atomes de carbone, en particulier de 9 à 30 atomes de carbone. IIs sont choisis plus particulièrement parmi l'acide myristique, l'acide palmitique, l'acide stéarique, l'acide béhénique, l'acide oléïque, l'acide linoléïque, l'acide linolénique et l'acide isostéarique,

Les esters sont les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂**-**C₂₂**,** linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou dioléate, stéarate, béhénate, oléopalmitate, linoléate, linolénate, oléostéarate, de saccharose, de glucose ou de méthylglucose.

On peut citer à titre d'exemple le produit vendu sous la dénomination GlucateⓇ DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft@ PSE.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1 m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. II s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONEⓇ 70045 V5 par RHODIA, ainsi que leurs mélanges.
   On peut également citer les cyclocopolymères du type diméthylsiloxanes/méthylalkylsiloxane, tel que la SILICONE VOLATILE^{®} FZ 3109 commercialisée par la société UNION CARBIDE, de formule : 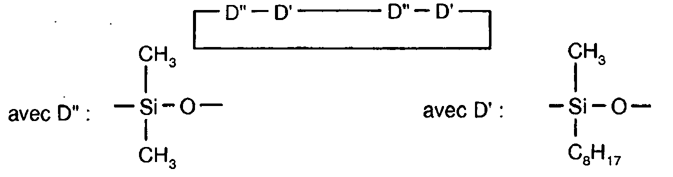 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25°C. II s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONEⓇ des séries 47 et 70 047 ou les huiles MIRASILⓇ commercialisées par RHODIA telles que, par exemple l'huile 70 047 V 500 000 ;
- les huiles de la série MIRASILⓇ commercialisées par la société RHODIA ;
- les huiles de la série 200 de la société DOW CORNING telles que la DC200 ayant viscosité 60 000 mm²/s ;
- les huiles VISCASILⓇ de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX@ 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/S et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RSiO_{3/2} et SIO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires t/ou ramifiés de viscosité allant de 1.10⁻⁵ à 5.1 0⁻²m²/S à 25 °C.

Parmi ces polyalkylarylsiloxanes on peut citer à titre d'exemple les produits commercialisés sous les dénominations suivantes :
- les huiles SILBIONEⓇ de la série 70 641 de RHODIA;
- les huiles des séries RHODORSILⓇ 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWETⓇ L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX@ 2428, 2434 et 2440 par la société GOLDSCHMIDT.

De préférence, le corps gras est un composé liquide à la température de 25°C et à la pression atmosphérique.

De préférence, le corps gras est choisi parmi l'huile de vaseline, les polydécènes, les esters liquides ou leurs mélanges.

La composition cosmétique anhydre présente une teneur en corps gras comprise avantageusement entre 10 et 99% en poids, de préférence entre 20 et 90% en poids, et encore plus particulièrement entre 25 et 80% en poids, par rapport au poids de la composition anhydre.

La composition anhydre cosmétique (A) comprend également un ou plusieurs tensioactifs.

De préférence, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

II est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les amines en C₈-C₃₀, saturées ou non, linéaires ou ramifiées, oxyalkylénées,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls ou en mélanges.

Les tensioactifs présentant un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, les amines en C₈-C₃₀, oxyéthylénées.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/Cₗ₂ à 1 mole de glycérol et l'alcool en C₁₂ à 1,5 mole de glycérol.

De préférence, le tensioactif présent dans la composition anhydre est un tensioactif non ionique.

La teneur en tensioactifs dans la composition anhydre, s'ils sont présents, représente plus particulièrement de 0,1 à 50 % en poids, de préférence de 0,5 à 30 % en poids par rapport au poids de la composition anhydre.

La composition cosmétique anhydre (A) peut également renfermer divers adjuvants utilisés classiquement dans les compositions pour la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition (A).

Selon une variante avantageuse de l'invention, la composition anhydre (A) comprend au moins une silice, de préférence à caractère hydrophobe, comme les silices pyrogénées.

Lorsqu'elles sont présentes, les silices représentent de 1 à 30 % en poids par rapport au poids de la composition anhydre (A).

Comme indiqué auparavant, la composition (B) mise en oeuvre dans le procédé selon l'invention comprend une ou plusieurs amines organiques dont le pKb à 25°C est inférieur à 12, et de préférence inférieur à 10, encore plus avantageusement inférieur à 6. II est à noter qu'il s'agit du pKb correspondant à la fonction de basicité la plus élevée.

Conformément à un mode de réalisation particulièrement avantageux de l'invention, l'amine organique est miscible au moins partiellement dans le ou les corps gras, à température ambiante et pression atmosphérique, présents dans la composition anhydre (A). De préférence, la ou les amines organiques sont totalement solubles à la température de 25°C et à la pression atmosphérique (760 mmHg) dans le ou les corps gras. De préférence, le ou les corps gras et la ou les amines organiques forment une phase unique à 25°C et à pression atmosphérique.

Selon une première variante de l'invention, l'amine organique comprend une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante : 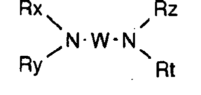 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆. On peut citer à titre d'exemple de telles amines, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante : 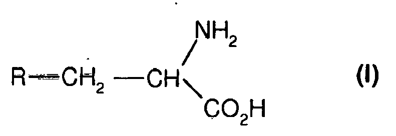 où R désigne un groupe choisi parmi :

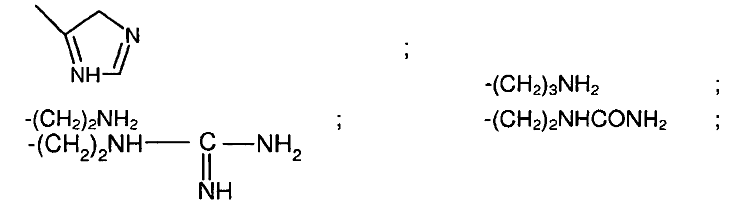

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Le ou les acides aminés, peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, les particules de poly(chlorure de vinylidène/acrylonitrile), notamment celles commercialisées sous la dénomination générale d'« Expancel^{®} » par la société AKZO NOBEL sous les références particulières « Expancel^{®} WE» ou « DE » Expancels, et leurs mélanges.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l'imidazole, le triazole, le tétrazole, le benzimidazole .

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique présente dans la composition anhydre est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

De manière avantageuse, la composition (B) présente une teneur en amine organique allant de 0,1 à 40% en poids, de préférence de 0,5 à 20% en poids par rapport au poids de ladite composition.

La composition (B) peut être une composition aqueuse ou non. Par composition aqueuse, on entend une composition comprenant plus de 5% en poids d'eau, de préférence plus de 10 % en poids d'eau, et de manière encore plus avantageuse plus de 20 % en poids d'eau.

De préférence, la composition (B) est une composition aqueuse.

Elle peut éventuellement comprendre un solvant organique. A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition (B), et de préférence de 5 à 30 % en poids.

Enfin, le procédé est mis en oeuvre avec une composition (C) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, et les peracides et leurs précurseurs.

Cet agent oxydant est avantageusement constitué par du peroxyde d'hydrogène et notamment en solution aqueuse (eau oxygénée) dont le titre peut varier, plus particulièrement, de 1 à 40 volumes, et encore plus préférentiellement de 5 à 40 volumes.

En fonction du degré d'éclaircissement souhaité, l'agent oxydant peut également comprendre un agent oxydant choisi de préférence parmi les sels peroxygénés.

De préférence, l'agent oxydant n'est pas choisi parmi les sels peroxygénés, les peracides et précurseurs.

La composition oxydante peut être aqueuse ou non. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition (C) est une composition aqueuse.

Elle peut également comprendre un ou plusieurs solvants organiques.

A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40% en poids par rapport au poids de la composition oxydante (C), et de préférence de 5 à 30 % en poids.

La composition oxydante peut comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante (C), lorsqu'elle est aqueuse, est inférieur à 7.

La composition oxydante (C) peut également renfermer d'autres ingrédients classiquement employés dans le domaine, comme notamment ceux détaillés auparavant dans le cadre de la anhydre cosmétique.

Enfin, la composition oxydante se présente sous diverses formes, comme par exemple une solution, une émulsion ou un gel.

Selon une première variante de l'invention, on applique sur les fibres kératiniques, sèches ou humides, une composition obtenue par mélange extemporané, au moment de l'emploi, de la composition anhydre (A), de la composition (B) et de la composition aqueuse oxydante (C) précitées.

Dans cette variante, les rapports pondéraux R1 des quantités de compositions (A) + (B)/(C) et R₂ des quantités de compositions (A)/(B) vont de 0,1 à 10, et de préférence de 0,3 à 3.

Conformément à une deuxième variante du procédé, on applique sur les fibres kératiniques, sèches ou humides, successivement et sans rinçage intermédiaire, les compositions (A), (B) et (C).

De préférence, on applique les compositions (A), puis (B), puis (C) ou (B) puis (A) puis (C).

Selon une autre variante, on peut également appliquer successivement et sans rinçage intermédiaire, la composition (C) puis le mélange résultant des compositions (A) et (B).

Dans ces deux variantes, les rapports pondéraux R₁ des quantités de compositions (A) + (B)/(C) et R₂ des quantités de compositions (A)/(B) vont plus particulièrement de 0,1 à 10, et de préférence de 0,3 à 3.

En outre, indépendamment de la variante mise en oeuvre, le mélange présent sur les fibres (résultant soit du mélange extemporané des compositions (A), (B) et (C) ou de leur application successive partielle ou totale) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées à sécher.

II est précisé que si la composition appliquée sur les cheveux (comprenant les compositions (A), (B) et (C)) comprenait de l'ammoniaque, sa teneur serait de préférence inférieure ou égale à 0,03 % en poids de la composition finale (exprimée en NH₃), plus particulièrement inférieure ou égale à 0,01 % en poids par rapport à la composition finale. II est indiqué que la composition finale résulte du mélange des compositions (A), (B) et (C) ; ces mélanges étant réalisés soit avant application sur les fibres kératiniques (préparation extemporanée) soit directement sur les fibres kératiniques (applications successives avec ou sans prémélanges et sans rinçage intermédiaire).

Mais les compositions (A), (B) et (C) ne comprennent de préférence pas d'ammoniaque.

Enfin, l'invention concerne un dispositif à plusieurs compartiments comprenant dans un premier compartiment une composition cosmétique anhydre (A), et dans un second, une composition aqueuse (C) comprenant un ou plusieurs agents oxydants également décrite auparavant, et dans un troisième, une composition (B) comprenant une ou plusieurs amines organiques dont le pKb à 25°C est inférieur à 12, ces compositions ayant été décrites auparavant.

Les exemples suivants servent à illustrer l'invention sans toutefois présenter un caractère limitatif.

### EXEMPLES

On prépare les compositions suivantes :

### Composition anhydre (A) : (les quantités sont exprimées en grammes)

| | |
|---|---|
| Mono-laurate de sorbitane oxyéthyléné (40E) | 21,67 |
| Silice pyrogénée à caractère hydrophobe | 11 |
| Huile vaseline | qsp 100 |

### Composition (B) comprenant 40 g de monoéthanolamine avec un complément à 100g d'eau.

Au moment de l'emploi, on mélange:
- 9 parties en poids de la composition anhydre (A)
- 1 partie en poids de la composition (B)
- 10 parties en poids de composition aqueuse oxydante comprenant 6% de peroxyde d'hydrogène à pH de 2,3 et comprenant environ 80% d'eau.

Le mélange résultant, dont le pH est d'environ 10, est appliqué sur une mèche châtain (hauteur de ton 5) naturelle.

Le temps de pause est de 30 minutes à température ambiante (environ 25°C).

A l'issue de ce temps de pause, la mèche est rincée, puis lavée avec du shampooing Elsève multivitamines.

A titre de comparaison, on prépare la composition suivante à base d'ammoniaque (quantités exprimées en g%) :

| | |
|---|---|
| Alcool oléïque polyglycérolé à 2 moles de glycérol | 4 |
| Alcool oléïque polyglycérolé à 4 moles de glycérol | 5,69 MA |
| Acide oléïque | 3 |
| Amine oléïque à 2 moles d'oxyde d'éthylène (ETHOMEEN 012 / Akzo) | 7 |
| Laurylamino succinamate de diéthylaminopropyle, sel de sodium (55% en matière active) | 3,0 MA |
| Alcool oléïque | 5 |
| Diéthanolamide d'acide oléïque | 12 |
| Alcool éthylique | 7 |
| Propylène glycol | 3,5 |
| Dipropylèneglycol | 0,5 |
| Monométhylether de propylèneglycol | 9 |
| Acétate d'ammonium | 0,8 |
| Ammoniaque à 20 % | 10 |
| Eau déminéralisée q.s.p. | 100 g |

| | |
|---|---|
| MA : quantité exprimée en matière active | |

Au moment de l'emploi, la composition comparative est mélangée poids pour poids avec une composition oxydante aqueuse comprenant 6 % de peroxyde d'hydrogène à pH de 2,3 et comprenant environ 80% d'eau.

Le mélange ainsi obtenu, dont le pH est d'environ 10, est ensuite appliqué sur une mèche châtain (hauteur de ton 5) naturelle.

Le temps de pause est de 30 minutes à température ambiante.

A l'issue de ce temps de pause, la mèche est rincée, puis lavée avec du shampooing Elsève multivitamines.

La composition selon l'invention ne dégage tout d'abord aucune odeur agressive, contrairement à la composition comparative qui dégage une forte odeur d'ammoniaque.

Le tableau ci-dessous montre que la composition selon l'invention, sans ammoniaque, donne un éclaircissement équivalent à celui de la composition comparative à base d'ammoniaque.

| | L* | ΔL* |
|---|---|---|
| Mèche châtain naturelle (HT5) non traitée | 19,82 | ---- |
| Composition de l'invention | 25,70 | 5,88 |
| Composition de l'état de la technique | 24,92 | 5,10 |

## Revendications

1. Procédé d'éclaircissement de fibres kératiniques humaines dans lequel on met en oeuvre :
a) une composition anhydre (A) comprenant un ou plusieurs corps gras et un ou plusieurs tensioactifs ;
b) une composition (B) comprenant une ou plusieurs amines organiques dont le pKb est inférieur à 12 ;
c) une composition (C) comprenant un ou plusieurs agents oxydants.

2. Procédé selon l'une quelconque des revendications précédentes, **caractérisé en ce que** le ou les corps gras sont choisis parmi les alcanes, les alcools gras, les acides gras, les esters d'acide gras, les esters d'alcool gras, les huiles minérales, végétales, animales ou synthétiques, les silicones, les cires.

3. Procédé selon l'une quelconque des revendications précédentes, **caractérisé en ce que** le ou les corps gras sont choisis parmi l'huile de vaseline, les polydécènes, les esters liquides ou leurs mélanges.

4. Procédé selon l'une quelconque des revendications précédentes, **caractérisé en ce que** la teneur en corps gras est comprise entre 10 et 99% en poids, par rapport au poids de la composition anhydre (A).

5. Procédé selon l'une quelconque des revendications précédentes, **caractérisé en ce que** la composition anhydre (A) comprend au moins un tensioactif non ionique, plus particulièrement choisi parmi les tensioactifs non ioniques mono- ou polyoxyalkylénés, mono- ou poly- glycérolés.

6. Procédé selon la revendication précédente, **caractérisé en ce que** la teneur en tensioactifs représente de 0,1 à 50% en poids, par rapport au poids de la composition anhydre (A).

7. Procédé selon l'une quelconque des revendications précédentes, **caractérisé en ce que** l'amine organique est miscible au moins partiellement dans le ou les corps gras.

8. Procédé selon l'une quelconque des revendications précédentes, **caractérisé en ce que** l'amine organique comprend une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

9. Procédé selon la revendication précédente, **caractérisé en ce que** l'amine organique est :
• une alcanolamine choisie parmi les mono-, di- ou tri- alcanolamine, comprenant un à trois radicaux hydroxyalkyle, identiques ou non, en C₁-C₄,
• un composé de formule suivante : 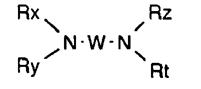 dans laquelle W est un reste alkylène en C₁-C₆ éventuellement substitué par un groupement hydroxyle ; Rx, Ry, Rz et Rt, identiques ou différents, représentent un atome d'hydrogène, un radical alkyle en C₁-C₆ ou hydroxyalkyle en C₁-C₆, un radical aminoalkyle en C₁-C₆.

10. Procédé selon l'une quelconque des revendications 8 ou 9, **caractérisé en ce que** l'amine organique est une alcanolamine, de préférence choisie parmi le 2-amino 2-méthyl 1-propanol, la monoéthanolamine ou leurs mélanges.

11. Procédé selon l'une quelconque des revendications précédentes, **caractérisé en ce que** l'amine organique est choisie parmi les acides aminés basiques de formule (i) suivante : 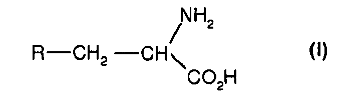 où R désigne un groupe choisi parmi :

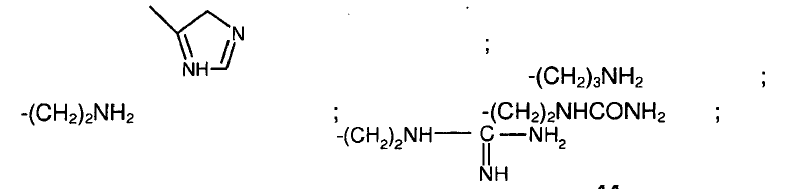

12. Procédé selon l'une quelconque des revendications 11, **caractérisé en ce que** l'amine organique est choisie parmi l'arginine, l'histidine, la lysine, ou leurs mélanges.

13. Procédé selon l'une quelconque des revendications précédentes, **caractérisé en ce que** la teneur en amine organique représente de 0,1 à 40% en poids, de préférence de 0,5 à 20% en poids par rapport au poids de la composition (B).

14. Procédé selon l'une quelconque des revendications précédentes, **caractérisé en ce que** l'on applique sur les fibres kératiniques, une composition obtenue par mélange extemporané, au moment de l'emploi, des compositions (A), (B) et (C).

15. Procédé selon l'une quelconque des revendications 1 à 13, **caractérisé en ce que** l'on applique sur les fibres kératiniques, successivement et sans rinçage intermédiaire, les compositions (A), (B) et (C), de préférence les compositions (A), puis (B), puis (C) ou (B) puis (A) puis (C).

16. Procédé selon l'une quelconque des revendications 1 à 13, **caractérisé en ce que** l'on applique sur les fibres kératiniques, successivement et sans rinçage intermédiaire, la composition (C) puis le mélange résultant des compositions (A) et (B).

17. Procédé selon l'une quelconque des revendications précédentes, **caractérisé en ce que** les rapports pondéraux R₁ des quantités de compositions (A) + (B)/(C) et R₂ des quantités de compositions (A)/(B) vont de 0,1 à 10.

18. Dispositif à plusieurs compartiments comprenant dans un premier compartiment, une composition anhydre (A) selon l'une des revendications 1 à 6, dans un autre compartiment une composition (B) telle que définie à l'une des revendications 7 à 13, et dans un troisième compartiment, une composition aqueuse comprenant un ou plusieurs agents oxydants.

## Patentansprüche

1. Verfahren zum Aufhellen von menschlichen Keratinfasern, bei dem man
(a) eine wasserfreie Zusammensetzung (A), die eine oder mehrere Fettsubstanzen und ein oder mehrere Tenside umfasst;
(b) eine Zusammensetzung (B), die ein oder mehrere organische Amine mit einem pKb-Wert von weniger als 12 umfasst;
(c) eine Zusammensetzung (C), die ein oder mehrere Oxidationsmittel umfasst;
verwendet.

2. Verfahren nach dem vorhergehenden Anspruch, **dadurch gekennzeichnet, dass** die Fettsubstanz bzw. die Fettsubstanzen aus Alkanen, Fettalkoholen, Fettsäuren, Fettsäureestern, Fettalkoholestern, Mineralöle, pflanzlichen Ölen, tierischen Ölen, synthetischen Ölen, Silikonen und Wachsen ausgewählt ist bzw. sind.

3. Verfahren nach einem der vorhergehenden Ansprüche, **dadurch gekennzeichnet, dass** die Fettsubstanz bzw. Fettsubstanzen aus Vaselineöl, Polydecenen, flüssigen Estern oder Mischungen davon ausgewählt ist bzw. sind.

4. Verfahren nach einem der vorhergehenden Ansprüche, **dadurch gekennzeichnet, dass** der Gehalt an Fettsubstanz zwischen 10 und 99 Gew.-%, bezogen auf das Gewicht der wasserfreien Zusammensetzung (A), liegt.

5. Verfahren nach einem der vorhergehenden Ansprüche, **dadurch gekennzeichnet, dass** die wasserfreie Zusammensetzung (A) mindestens ein nichtionisches Tensid, das insbesondere aus mono- oder polyoxyalkylenierten, mono- oder polyglycerinierten nichtionischen Tensiden ausgewählt ist, umfasst.

6. Verfahren nach dem vorhergehenden Anspruch, **dadurch gekennzeichnet, dass** der Gehalt an Tensiden 0,1 bis 50 Gew.-%, bezogen auf das Gewicht der wasserfreien Zusammensetzung (A), ausmacht.

7. Verfahren nach einem der vorhergehenden Ansprüche, **dadurch gekennzeichnet, dass** das organische Amin zumindest teilweise in der Fettsubstanz bzw. den Fettsubstanzen mischbar ist.

8. Verfahren nach einem der vorhergehenden Ansprüche, **dadurch gekennzeichnet, dass** das organische Amin eine oder zwei primäre, sekundäre oder tertiäre Aminfunktionen und eine oder mehrere lineare oder verzweigte C₁-C₈-Alkylgruppen mit einem oder mehreren Hydroxylresten umfasst.

9. Verfahren nach dem vorhergehenden Anspruch, **dadurch gekennzeichnet, dass** es sich bei dem organischen Amin um
• ein Alkanolamin, ausgewählt aus Mono-, Di- und Trialkanolaminen, mit einem bis drei gleichen oder verschiedenen C₁-C₄-Hydroxyalkylresten,
• eine Verbindung der folgenden Formel : 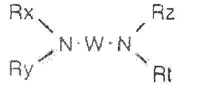 worin W für einen gegebenenfalls durch eine Hydroxylgruppe substituierten C₁-C₆-Alkylenrest steht; Rx, Ry, Rz und Rt gleich oder verschieden sind und für ein Wasserstoffatom oder einen C₁-C₆-Alkyl-, C₁-C₆-Hydroxyalkyl- oder C₁-C₆-Aminoalkyl-rest stehen;
handelt.

10. Verfahren nach einem der Ansprüche 8 oder 9, **dadurch gekennzeichnet, dass** es sich bei dem organischen Amin um ein Alkanolamin, vorzugsweise ausgewählt aus 2-Amino-2-methyl-1-propanol und Monoethanolamin oder Mischungen davon, handelt.

11. Verfahren nach einem der vorhergehenden Ansprüche, **dadurch gekennzeichnet, dass** das organische Amin aus den basischen Aminosäuren der folgenden Formel (I): 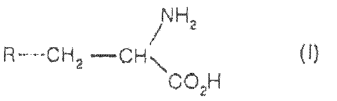 worin R für eine aus 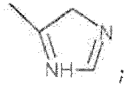 - (CH₂)₃NH₂ ;
- (CH₂) ₂NH₂; - (CH₂) ₂NHCONH₂ ; 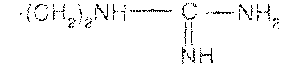 ausgewählte Gruppe steht, ausgewählt ist.

12. Verfahren nach dem Anspruch 11, **dadurch gekennzeichnet, dass** das organische Amin aus Arginin, Histidin, Lysin oder Mischungen davon ausgewählt ist.

13. Verfahren nach einem der vorhergehenden Ansprüche, **dadurch gekennzeichnet, dass** der Gehalt an organischem Amin 0,1 bis 40 Gew.-% und vorzugsweise 0,5 bis 20 Gew.-%, bezogen auf das Gewicht der Zusammensetzung (B), ausmacht.

14. Verfahren nach einem der vorhergehenden Ansprüche, **dadurch gekennzeichnet, dass** man eine durch unmittelbares Mischen der Zusammensetzungen (A), (B) und (C) zum Zeitpunkt der Anwendung erhaltene Zusammensetzung auf die Keratinfasern aufbringt.

15. Verfahren nach einem der Ansprüche 1 bis 13, **dadurch gekennzeichnet, dass** man die Zusammensetzungen (A), (B) und (C), vorzugsweise die Zusammensetzungen (A), dann (B) und dann (C) oder (B), dann (A) und dann (C), nacheinander und ohne Zwischenspülung auf die Keratinfasern aufbringt.

16. Verfahren nach einem der Ansprüche 1 bis 13, **dadurch gekennzeichnet, dass** man die Zusammensetzung (C) und dann die aus den Zusammensetzungen (A) und (B) erhaltene Mischung nacheinander und ohne Zwischenspülung auf die Keratinfasern aufbringt.

17. Verfahren nach einem der vorhergehenden Ansprüche, **dadurch gekennzeichnet, dass** die Gewichtsverhältnisse R₁ der Mengen der Zusammensetzungen (A) + (B)/(C) und R₂ der Mengen der Zusammensetzungen (A)/(B) 0,1 bis 10 betragen.

18. Vorrichtung mit mehreren Kompartimenten, das in einem ersten Kompartiment eine wasserfreie Zusammensetzung (A) nach einem der Ansprüche 1 bis 6, in einem anderen Kompartiment eine Zusammensetzung (B) gemäß einem der Ansprüche 7 bis 13 und in einem dritten Kompartiment eine wässrige Zusammensetzung, die ein oder mehrere Oxidationsmittel umfasst, umfasst.

## Claims

1. Process for lightening human keratin fibres, in which the following are used:
(a) an anhydrous composition (A) comprising one or more fatty substances and one or more surfactants;
(b) a composition (B) comprising one or more organic amines whose pKb is less than 12;
(c) a composition (C) comprising one or more oxidizing agents.

2. Process according to the preceding claim, **characterized in that** the fatty substance(s) is (are) chosen from alkanes, fatty alcohols, fatty acids, fatty acid esters, fatty alcohol esters, mineral oils, plant oils, animal oils, synthetic oils, silicones and waxes.

3. Process according to either of the preceding claims, **characterized in that** the fatty substance(s) is (are) chosen from liquid petroleum jelly, polydecenes and liquid esters, or mixtures thereof.

4. Process according to any one of the preceding claims, **characterized in that** the fatty substance content is between 10% and 99% by weight relative to the weight of the anhydrous composition (A).

5. Process according to any one of the preceding claims, **characterized in that** the anhydrous composition (A) comprises at least one nonionic surfactant more particularly chosen from monooxyalkylenated or polyoxyalkylenated, monoglycerolated or polyglycerolated nonionic surfactants.

6. Process according to the preceding claim, **characterized in that** the surfactant content represents from 0.1% to 50% by weight relative to the weight of the anhydrous composition (A).

7. Process according to any one of the preceding claims, **characterized in that** the organic amine is at least partially miscible in the fatty substance(s).

8. Process according to any one of the preceding claims, **characterized in that** the organic amine comprises one or two primary, secondary or tertiary amine functions, and one or more linear or branched C₁-C₈ alkyl groups bearing one or more hydroxyl radicals.

9. Process according to the preceding claim, **characterized in that** the organic amine is:
• an alkanolamine chosen from mono-, di- and trialkanolamines, comprising from one to three identical or different C₁-C₄ hydroxyalkyl radicals,
• a compound having the following formula: 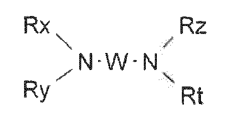 in which W is a C₁-C₆ alkylene residue optionally substituted with a hydroxyl group; Rx, Ry, Rz and Rt, which may be identical or different, represent a hydrogen atom or a C₁-C₆ alkyl, C₁-C₆ hydroxyalkyl or C₁-C₆ aminoalkyl radical.

10. Process according to either of Claims 8 and 9, **characterized in that** the organic amine is an alkanolamine, preferably chosen from 2-amino-2-methyl-1-propanol and monoethanolamine, or mixtures thereof.

11. Process according to any one of the preceding claims, **characterized in that** the organic amine is chosen from the basic amino acids of formula (I) below: 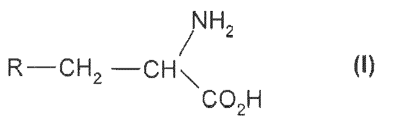 in which R denotes a group chosen from: 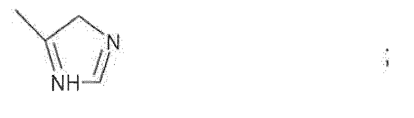 - (CH₂) ₃NH₂ ; - (CH₂) ₂NH₂ ; - (CH₂) ₂NHCONH₂ ;

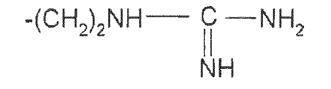

12. Process according to Claim 11, **characterized in that** the organic amine is chosen from arginine, histidine and lysine, or mixtures thereof.

13. Process according to any one of the preceding claims, **characterized in that** the organic amine content represents from 0.1% to 40% by weight and preferably from 0.5% to 20% by weight relative to the weight of composition (B).

14. Process according to any one of the preceding claims, **characterized in that** a composition obtained by extemporaneous mixing, at the time of use, of compositions (A), (B) and (C) is applied to the keratin fibres.

15. Process according to any one of Claims 1 to 13, **characterized in that** compositions (A), (B) and (C), preferably compositions (A) and then (B) and then (C), or (B) and then (A) and then (C), are applied to the keratin fibres, successively and without intermediate rinsing.

16. Process according to any one of Claims 1 to 13, **characterized in that** composition (C) and then the mixture resulting from compositions (A) and (B) are applied to the keratin fibres, successively and without intermediate rinsing.

17. Process according to any one of the preceding claims, **characterized in that** the weight ratios R1 of the amounts of compositions (A) + (B) / (C) and R2 of the amounts of compositions (A)/(B) range from 0.1 to 10.

18. Multi-compartment device comprising, in a first compartment, an anhydrous composition (A) according to one of Claims 1 to 6, in another compartment, a composition (B) as defined in one of Claims 7 to 13, and, in a third compartment, an aqueous composition comprising one or more oxidizing agents.
